**Europäisches Patentamt**

**European Patent Office**

**Office européen des brevets**

(19)

(11) Veröffentlichungsnummer : **0 329 605 B1**

(12) **EUROPÄISCHE PATENTSCHRIFT**

(45) Veröffentlichungstag der Patentschrift :
**29.01.92 Patentblatt 92/05**

(51) Int. Cl.$^5$ : **A61K 37/02,** A61K 35/16

(21) Anmeldenummer : **89810093.8**

(22) Anmeldetag : **03.02.89**

(54) Verfahren zur Herstellung von Apolipoproteinen aus Blutplasma- oder Serumfraktionen.

(30) Priorität : **08.02.88 CH 431/88**

(43) Veröffentlichungstag der Anmeldung :
**23.08.89 Patentblatt 89/34**

(45) Bekanntmachung des Hinweises auf die
Patenterteilung :
**29.01.92 Patentblatt 92/05**

(84) Benannte Vertragsstaaten :
**AT BE CH DE ES FR GB GR IT LI LU NL SE**

(56) Entgegenhaltungen :
**AT-B- 380 167
FR-A- 2 343 251
JOURNAL OF AMERICAN CHEMICAL SO-
CIETY, Band 68, 1946, Seiten 459-475; E.J.-
COHN et al.: "Preparation and properties of
serum and plasma proteins. IV. A system for
the separation into fractions of the protein and
lipoprotein components of biological tissues
and fluids"
JOURNAL OF CHROMATOGRAPHY, Band 414,
1987, Seiten 35-45; Elsevier Sc. Publ. B.V.,
Amsterdam, NL H.MEZDOUR et al.:
"Anion-exchange fast protein liquid chromatographic characterization and purification of
apolipoproteins A-I, A-II, C-I, C-II, C-IIIO, C-III1,
C-III2, and E from human plasma"**

(73) Patentinhaber : **ROTKREUZSTIFTUNG
ZENTRALLABORATORIUM
BLUTSPENDEDIENST SRK
Wankdorfstrasse 10
CH-3000 Bern 22 (CH)**

(72) Erfinder : **Isliker, Henri
16, Chemin Languedoc
CH-1007 Lausanne (CH)**
Erfinder : **Peitsch, Manuel Claude
610 West Patrick Street
Apartment 3 Frederick MD 21701 (US)**
Erfinder : **Heiniger, Hans-Jörg
Altenbergstrasse 126
CH-3013 Bern (CH)**
Erfinder : **Lerch, Peter Georg
Rosenweg 20
CH-3007 Bern (CH)**
Erfinder : **Morgenthaler, Jean-Jacques
Aeschiweg 9
CH-3067 Boll (CH)**

(74) Vertreter : **Frossard, Michel, Dr. et al
A. Braun, Braun, Héritier, Eschmann AG,
Patentanwälte Holbeinstrasse 36-38
CH-4051 Basel (CH)**

EP 0 329 605 B1

## Beschreibung

Die Ursachen der heute sehr häufigen kardiovaskulären Erkrankungen sind komplex ; immerhin wurden verschiedene Risikofaktoren definiert, die mit diesen Erkrankungen einhergehen. Weist ein Individuum ein oder sogar mehrere dieser Faktoren auf, so besteht für ihn/sie ein erhöhtes Risiko von kardiovaskulären Erkrankungen. Zu diesen Risikofaktoren gehören : erhöhter Blutdruck ; Rauchen (besonders Zigaretten) ; Übergewicht ; Hypercholesterinämie ; männliches Geschlecht ; Diabetes ; Häufung von kardiovaskulären Erkrankungen in der Familie.

Gewisse Risiken sind unvermeidlich ; andere können durch Verhaltensänderungen beeinflusst werden ; auf dritte kann durch Medikamente eingewirkt werden. Zu der letzteren Gruppe gehört das Risiko, welches durch einen erhöhten Cholesterinspiegel im Blut dargestellt wird. Zunächst muss zwischen zwei verschiedenen Komponenten unterschieden werden, welche beide Cholesterin enthalten : den sogenannten *low density lipoproteins* (LDL ; Lipoproteine niederer Dichte) und den *high density lipoproteins* (HDL ; Lipoproteine hoher Dichte). Während Cholesterin, welches in der LDL-Fraktion auftritt, das Risiko kardiovaskulärer Krankheiten erhöht ("schlechtes Cholesterin"), erniedrigt umgekehrt das HDL-assoziierte Cholesterin dieses Risiko ("gutes Cholesterin").

Neben den erwähnten HDL und LDL treten im Blut auch Chylomikronen auf, sowie *very low density lipoproteins* (VLDL ; Lipoproteine sehr niederer Dichte) und *intermediate density lipoproteins* (IDL ; Lipoproteine intermediärer Dichte). Die HDL lassen sich in die Untergruppen $HDL_1$, $HDL_2$ und $HDL_3$ unterteilen. Die Unterscheidungen in Gruppen und Untergruppen erfolgen aufgrund von verschiedenen physikochemischen (z.B. Dichte, Zusammensetzung, Morphologie) und funktionellen Eigenschaften.

Es sind Medikamente beschrieben worden, welche die Cholesterinkonzentration im Blut generell erniedrigen, oder sogar spezifisch die Konzentration des LDL-Cholesterins erniedrigen, diejenige des HDL-Cholesterins dagegen erhöhen. Diese Therapien beruhen auf verschiedenen Wirkungsmechanismen : einerseits gelingt es, die Aufnahme von Lipiden im Darm zu erniedrigen ; andererseits kann man auch ein Schlüsselenzym der Cholesterinbiosynthese (Hydroxymethylglutaryl-CoA-Reduktase ; HMG-CoA-Reduktase) hemmen und dadurch die Syntheserate erniedrigen. Alle diese Medikamente (Colestipol, Nikotinsäure, Lovastatin usw.) müssen aber über lange Zeit eingenommen werden, so dass mögliche Nebenwirkungen nicht ausgeschlossen werden können.

HDL sind komplexe Gebilde, welche aus Lipiden (u.a. Phosphatidylcholin, Sphingomyelin, Cholesterin und Cholesterinestern) und Proteinen (Apoproteine : u.a. ApoA-I, APoA-II, Apo E) zusammengesetzt sind. Die einzelnen Bestandteile sind durch nichtkovalente Bindungen zusammengehalten ; HDL unterliegt aber einem ständigen Metabolismus : einzelne Komplexe werden zerlegt und wiederum aus anderen Komponenten zusammengebaut. Daraus ergibt sich eine weitere Möglichkeit, das Verhältnis von LDL- zu HDL-Cholesterin zugunsten des "guten Cholesterins" zu verändern : durch Zugabe des im HDL vorkommenden Proteins (der Proteine) kann das Gleichgewicht verschoben werden, so dass mehr Cholesterin ins HDL eingebaut wird.

Aus Blutplasma sind die erwünschten, biologisch günstigen Lipoproteine hoher Dichte (HDL) auf konventionelle Art isoliert und als Substitutionstherapie zur Behandlung der familiären An-alpha-lipoproteinämie (Tangier-Krankheit) bereits vorgeschlagen worden.

Zur Isolierung dieser HDL hat man bisher Blutplasma einer Reihe von aufeinanderfolgenden Flotationsvorgängen bei verschiedenen Dichtewerten unterworfen [H.B. Brewer, Methods in Enzymology **128** ; J.P. Segrest, J.J. Albers, Herausgeber ; Academic Press, Orlando, FL, U.S.A., 1986.]. Dabei wurde Plasma durch Versetzen mit festem Kaliumbromid auf eine bestimmte Dichte eingestellt und einer ersten Ultrazentrifugation unterworfen ; die obere Schicht wurde abgetrennt und die untere wiederum mit Kaliumbromid oder einer konzentrierten Kaliumbromidlösung versetzt und das Gemisch einer zweiten Ultrazentrifugation unterworfen. Auf diese Art konnte eine Fraktionierung erwirkt werden, bei welcher zuerst die Lipoproteine niederer Dichte, später die Lipoproteine hoher Dichte erhalten wurden.

Ein solches Verfahren eignet sich allerdings für eine industrielle Anwendung überhaupt nicht, weil das Fassungsvermögen der bisher erhältlichen Ultrazentrifugen höchstens bei 400 mL liegt ; die Ausbeute des langwierigen und umständlichen Verfahrens beträgt dementsprechend bestenfalls einige hundert mg. Zudem kann das bei dieser Methode eingesetzte Plasma nicht mehr mit den etablierten Fraktionierverfahren zur Isolierung anderer, wertvoller Komponenten verwendet werden.

Zur Abtrennung der Lipoproteinfraktionen aus menschlichem Serum und gegebenenfalls zu ihrer Bestimmung wird gemäss AT-B-380167 eine fraktionierte Fällung mittels Polyethylenglykol durchgeführt, wobei je nach Polyethylenglykolkonzentration und pH-Wert des Mediums die VLDL- und LDL-Fraktionen im Niederschlag und die $HDL_2$- und $HDL_3$-Fraktionen im Überstand, bzw. die VLDL-, LDL- und $HDL_2$-Fraktionen im Niederschlag und die $HDL_3$-Fraktion im Überstand erhalten werden.

Gemäss diesem Verfahren erhaltene Fraktionen, Niederschläge sowie auch Überstände sind mehr oder weniger stark mit Polyethylenglykol kontaminiert, welches sich nur noch schwer von den Porteinen abtrennen lässt.

Nach einem früheren Verfahren gemäss FR 2343251 werden die Lipoproteine von den andern Blutproteinen durch Ionenaustauschchromatographie an einem Kationenaustauscher isoliert. Unter geeigneten Bedingungen werden die VLDL- und die LDL-Fraktion an den Ionentauscher gebunden, während die HDL-Fraktion durch die Säule hindurchläuft. Die VLDL- und LDL-Fraktionen können durch Ändern der Lösungsbedingungen eluiert und gewonnen werden. Die HDL-Fraktion kann mit einem andern Kationenaustauscher ebenfalls gebunden und anschliessend eluiert werden.

Harnstofflösliche LDL- und HDL-Apolipoproteine können ebenfalls mittels Anionenaustauschern in einzelne Komponenten aufgetrennt werden [H. Mezdour und Mitarb., J. Chromatogr. **414**, 35-45, 1987]. Besonders geeignet ist dazu das FPLC (*fast protein liquid chromatography*) System der Firma Pharmacia, Uppsala, Schweden, wenn mit der Mono Q Säule gearbeitet wird. Die Trennung kann dann in 30 Minuten durchgeführt werden. Zum Erzielen einer genügenden Reinheit ist eine zweit Reinigungsstufe erforderlich.

Chromatographische Verfahren zu Trennung von Proteinen sind im Laboratorium weit verbreitet ; zur Gewinnung von Porteinen im industriellen Massstab sind sie oft weniger geeignet, weil sie zu teuer sind und/oder nur die Trennung von kleinen Mengen erlauben. Dazu kommt, dass das Material vor der Trennung auf eine Proteinkonzentration von etwa 2% eingestellt, im Fall von Plasma also verdünnt werden muss. Dieses verdünnte Plasma kann dann nicht mehr wie bisher durch die im nächsten Abschnitt erwähnten Verfahren fraktioniert werden.

Andererseits wird Blutplasma heute in industriellem Massstab gewonnen und in einzelne Fraktionen aufgetrennt, darunter insbesondere solche, welche die Lipoproteine enthalten. Diese Fraktionen können nach einem ursprünglich in den Vereinigten Staaten entwickelten, als Cohn-Oncley-Methode bekannten Verfahren [E.J. Cohn und Mitarb., J. Am. Chem. Soc. **68**, 459-475, 1946 ; J.L. Oncley und Mitarb, J. Am. Chem. Soc. **71**, 541-550, 1949] gewonnen werden. Die Lipoproteine enthaltenden Fraktionen können ebenfalls nach einer Variante dieses Verfahrens, der Kistler-Nitschmann-Methode [P. Kistler und H. Nitschmann, Vox Sang. **7**, 414-424, 1962 ; H. Nitschmann und Mitarb., Helv. Chim. Acta **37**, 866-873, 1954] erhalten werden.

Es wurde nun gefunden, dass man aus den erwähnten, die Lipoproteine enthaltenden Fraktionen die gewünschten HDL-Apolipoproteine nach einem bedeutend einfacheren und industriell anwendbaren Verfahren herstellen kann. Das Herstellungsverfahren lässt sich problemlos in die bestehenden Fraktionierverfahren integrieren, da es von Fraktonen ausgeht, die bisher nicht weiter verarbeitet wurden und als Abfall eliminiert werden mussten.

Erfindungsgemäss werden die Apolipoproteine aus Fraktionen von menschlichem Blutplasma oder Serum erhalten, welche Lipoproteine enthalten, indem man besagte Fraktionen in einer wässerigen Pufferlösung im pH-Bereich 3 bis 5 oder 6 bis 9 suspendiert und zur Ausfällung von unerwünschten Begleitstoffen mit einem niederen aliphatischen Alkohol (beispielsweise Methanol, Ethanol, Propanol oder Isopropanol ; vorzugsweise Ethanol) inkubiert ; die Lipoproteine enthaltende Phase wird wiederum abgetrennt und die darin enthaltenen Lipoproteine werden aufkonzentriert. Falls sich, beispielsweise als Folge einer Präzipitation beim Aufkonzentrieren, Apolipoproteinaggregate gebildet haben, können diese durch Auflösen bei erhöhter Temperatur, bei leicht basischem pH oder mittels Zusatz von chaotropen Stoffen oder Tensiden desaggregiert werden.

Das Ausgangsmaterial wird in einer wässerigen Pufferlösung suspendiert, welche vorzugsweise einen pH-Wert im Bereich von 3 bis 5 oder 6 bis 9 aufweist. Als Puffersubstanzen können beispielsweise $NaHCO_3$, $Na_2B_4O_7$, $NA_2HOP_4$, Natriumcitrat oder Tris, nach entsprechender Einstellung des pH-Wertes, verwendet werden ; die Konzentrationen dieser Substanzen liegen im Bereich 5 mM bis 2 M. Zur Förderung der Auflösung bzw. Extraktion können chaotrope Stoffe, z.B. Harnstoff, Guanidinhydrochlorid oder ein anorganisches wasserlösliches Thiocyanat wie insbesondere Ammoniumthiocyanat, zugegeben werden, wonach eine Extraktion der Apolipoproteine im pH-Bereich von 3 bis 9 möglich ist.

Zum selben Zweck können auch Tenside verwendet werden, z.B. Polysorbate wie insbesondere das Polyoxyethylen-(20)-sorbitan-monolaurat, -monopalmitat, -monostearat oder -monooleat (Polysorbat 20, 40, 60 oder 80 bzw. die als Tween® von entsprechender Nummer bekannten Produkte der ICI America Inc., Atlas Chemicals Division, Wilmington, DE, USA) oder ein Octylphenoxy-polyethoxyethanol (Triton® X- 45, X-100 oder X-114 von Rohm and Haas Co., Philadelphia, PA, USA) oder auch Verbindungen wie die Polyethylenglykole, z.B. Polyethylenglykol 4000.

Zur Förderung des Übergangs in Lösung können ebenfalls wasserlösliche Salze, z.B. Natriumchlorid, in einem Konzentrationsbereich von bis zu 5 Mol/L zugesetzt werden.

Die Extraktion der Apolipoproteine kann auch unter Zusatz eines vorzugsweise apolaren organischen Lösungsmittels wie Chloroform, Dichlormethan, Hexan oder Benzol vorgenommen werden, wobei die Apolipoproteine in die wässerige Phase übergehen.

Diejenige Phase, die beim Suspendieren des Ausgangsmaterials die Apolipoproteine enthält, wird abgetrennt, vorzugsweise durch Filtrieren, Zentrifugieren oder Dekantieren, während die andern Phasen verworfen werden. Die Identifikation der Phase, welche die Apolipoproteine enthält, erfolgt durch geeignete analytische Methoden, beispielsweise durch Polyacrylamidgel-Elektrophorese.

Zur Ausfällung unerwünschter Begleitstoffe wird die abgetrennte Phase, vorzugsweise in der Kälte, mit einem niederen, aliphatischen Alkohol (beispielsweise Methanol, Ethanol, Propanol oder Isopropanol; vorzugsweise Ethanol) versetzt und inkubiert. Hierauf wird die Fällung, mit Vorteil durch Filtrieren oder Zentrifugieren, abgetrennt und aus dem Überstand werden Ethanol und andere unerwünschte Begleitstoffe durch die weiter unten angegebenen Reinigungsmassnahmen entfernt.

Auch kann die Extraktion der Apolipoproteine mittels der wässerigen Pufferlösung gleichzeitig mit der Ausfällung unerwünschter Begleitstoffe durch den niederen aliphatischen Alkohol, in demselben Arbeitsgang, durchgeführt werden.

Im letzteren Fall kann das bereits erwähnte apolare organische Lösungsmittel ebenfalls zugesetzt werden. Es bildet sich dann ein Mehrphasensystem, von welchem die wässerige Phase abgetrennt und nochmals mit dem niederen aliphatischen Alkohol inkubiert wird.

Die Reinigung der im Überstand vorliegenden Apolipoproteine kann nach verschiedenen Methoden durchgeführt werden. Eine erste Möglichkeit besteht darin, dass der Überstand gegen eine wässerige Pufferlösung im pH-Bereich 3 bis 5 oder 6 bis 9 dialysiert wird ; bei der Dialyse bleiben die Apolipoproteine in Lösung, noch vorhandene Verunreinigungen fallen grösstenteils aus und können abfiltriert werden.

Nach einer anderen Methode wird der Überstand mit einer wässerigen Pufferlösung (beispielsweise 10 mM Natriumphosphat, pH 8, mit 150 mM NaCl) im pH-Bereich um 8 versetzt. Es entsteht eine Fällung, welche verworfen wird, während der Überstand die Apolipoproteine enthält und weiter verarbeitet werden kann.

Aus Lösungen, welche gereinigte Apolipoproteine enthalten, können diese durch verschiedene Methoden aufkonzentriert werden, beispielsweise durch Membranfilration oder durch Lyophilisation. Sie können aber auch durch Einstellen des pH-Wertes auf 5 bis 6 und/oder des Gehaltes an Natriumchlorid oder einem anderen wasserlöslichen Salz auf eine Konzentration von 0.1 bis 0.2 Mol/L ausgefällt werden. Die Apolipoproteine können ebenfalls durch Erhöhung der Alkoholkonzentration (vorzugsweise Ethanol) bis auf 80% oder durch Zusatz von Polymeren wie Polyvinylpyrrolidon oder Dextransulfat ausgefällt werden. Sie können auch an Ionentauscher, beispielsweise den Anionentauscher DEAE-Trisacryl LS gebunden und anschliessend durch Erhöhen der Ionenstärke mit einem wasserlöslichen Salz, beispielsweise $NH_4HCO_3$, wieder (in höherer Konzentration) eluiert werden.

Ferner lässt sich im Laufe des Verfahrens, auf einer beliebigen Stufe, eine Entfernung der Lipide vornehmen, indem man das Produkt mit einem vorzugsweise apolaren organischen Lösungsmittel, z.B. einem aliphatischen Kohlenwasserstoff wie Hexan, einem aliphatischen halogenierten Kohlenwasserstoff wie Chloroform oder Dichloromethan, einem Ether wie Diethylether oder Dibutylether oder einem Gemisch von solchen Lösungsmitteln extrahiert. Die Entfernung der Lipide kann insbesondere am Anfang des Verfahrens, gleichzeitig mit dem Suspendieren in einer wässerigen Pufferlösung, oder ganz am Schluss, mit dem lyophilisierten Produkt stattfinden.

Zur Solubilisierung des präzipitierten Apolipoproteins und zur Überführung in ein aggregatfreies Produkt können bei der Auflösung chaotrope Substanzen wie Guanidin oder Harnstoff, oder ionische oder nichtionische Detergenzien, oder Kombinationen davon zugegeben werden, die anschliessend mittels einer geeigneten Methode wie Dialyse, Diafiltration, Adsorption oder Gelfiltration wieder entfert werden, oder die Solubilisierung kann in einem leicht basischen Puffer wie beispielsweise Tris, Phosphat oder Karbonat bei Temperaturen bis zu 70°C durchgeführt werden. Die Qualität des Produktes bezüglich Stabilität, Sterilität und Virussicherheit kann durch Pasteurisation, vorzugsweise im Endbehälter in Gegenwart eines Stabilisators (beispielsweise eines Kohlehydrates wie Saccharose oder Mannit) noch erhöht werden.

Zur Anwendung am Menschen müssen die Apolipoproteine in eine geeignete galenische Form überführt werden. Eine wässerige Losung von erfindungsgemäss gewonnenen Apolipoprotein, mit oder ohne Zusatz von geeigneten Puffer substanzen (beispielsweise Natriumphosphat, -karbonat oder -zitrat im physiologischen pH-Bereich) kann verwendet werden. Die Lösung kann aber auch nach Herstellung lyophilisiert werden und vor Gebrauch in einem geeigneten Lösungsmittel (Wasser, Pufferlösung oder Salzlösung, beispielsweise NaCl) aufgelöst werden. Es ist ebenfalls möglich, mit den Apolipoproteinen und mit Lipiden (Phospholipide wie Leicithin, allein oder zusammen mit anderen Lipiden wie Cholesterin) Proteoliposomen herzustellen und medizinisch einzusetzen.

Ausgehend vom Niederschlag B einer Kistler-Nitschmann Fraktionierung wurden Ausbeuten von bis zu 70% des im Niederschlag B vorliegenden Apolipoproteins A-I erzielt, bei Isolierungsversuchen mit Vorfällung IV wurden Ausbeuten von bis zu 50% erzielt, wobei Reinheiten von 90 bis 95% erreicht wurden. HDL enthält hauptsächlich die Apolipoproteine A-I, A-II und A-IV ; bei der Fraktionierung nach Kistler und Nitschmann findet man je etwa 40% der im Ausgangsplasma enthaltenen Menge Apolipoprotein A-I im Niederschlag B und in der Vorfällung IV.

Reinheiten und Ausbeute wurden mittels SDS-Polyacrylamidgel-Elektrophorese und Proteinbestimmungsmethoden ($A_{280\ nm}$, Folin und BioRad Protein Assay [BioRad Laboratories, München, BRD]) bestimmt.

Apolipoproteine, besonders das Apolipoprotein A-I (ApoA-I), sind befähigt, die im menschlichen Blut vorliegende Lecithin-Cholesterin-Acyltransferase (LCAT) zu aktivieren. Die erfindungsgemäss erhältlichen Apolipoproteine enthalten vorwiegend ApoA-I und aktivieren LCAT in hohem Masse. Zur in vitro Prüfung eignet sich $^{14}C$-markiertes Cholesterin, das in Gegenwart von Lecithin und Apolipoprotein in $^{14}C$-Cholesterinester übergeführt wird und mit Hilfe von Dünnschichtchromatographie nachgewiesen werden kann.

Die Bedeutung des neuen Verfahrens liegt unter anderem darin, dass nicht nur Apolipoproteine aus Plasmafraktionen, die bisher als Abfallprodukt verworfen wurden, industriell hergestellt werden können, sondern dass diese Produkte auch nach Lagerung ihre biologische Aktivität beibehalten.

Die nach dem erfindungsgemässen Verfahren erhaltenen Apolipoproteine können insbesondere zur Prophylaxe und Therapie von kardiovaskulären Krankheiten bzw. zur Herstellung von dafür bestimmten pharmazeutischen Präparaten verwendet werden. Sie eignen sich vor allem zur Behandlung — ob prophylaktisch oder kurativ — der Hypercholesterinämie jeder Genese und bei all deren Erscheinungsformen. Sie können dabei als alleiniger Wirkstoff oder auch in Kombination mit Aktivatoren der Lecithin-Cholesterin-Acyltransferase (LCAT) und deren Substrate, vor allem Lecithin, zur Anwendung kommen. Ferner können sie ebenfalls als Substitutionstherapie bei der LDL-Apherese und in Kombination mit Inhibitoren der Hydroxymethylglutaryl-CoA-Reduktase eingesetzt werden. Schliesslich können sie als postoperative prophylaktische Infusion nach Herztransplantation, Tripelbypass- und anderen Herzoperationen verwendet werden.

Ein weiterer Gegenstand der Erfindung ist also die Herstellung pharmazeutischer Präparate, welche die verfahrensgemäss hergestellten Apolipoproteine als Wirkstoff nebst den üblichen Trägerstoffen und Excipientien enthalten und zur Prophylaxe und Therapie von kardiovaskulären Krankheiten, vor allem der Hypercholesterinämie und bei den anderen zuvor erwähnten Indikationen verwendet werden sollen.

Die Verabreichung der Apolipoproteine bei den angegebenen therapeutischen Indikationen soll intravenös, durch intravenöse Injektion oder Infusion, oder peroral oder rektal erfolgen. Als Posologie wird beim erwachsenen Menschen eine Dosis von 1 mg bis 1 g/(kg Körpergewicht × Tag) empfohlen.

BEISPIEL 1

0.5 g zentrifugierter Niederschlag B [Kistler-Nitschmann Verfahren] wurde in 1 mL Puffer (10 mM Tris/HCl [Tris(hydroxymethyl)-aminomethan Hydrochlorid], pH 8.0, 6 M Harnstoff) suspendiert; nach Zugabe von 1 mL Chloroform/Ethanol (volumenverhältnis 1 : 1) wurde gründlich gemischt und 1 Stunde bei 0°C inkubiert. Anschliessend wurde während 10 Minuten bei 0°C und 10'000 × g zentrifugiert. Die Unter- und die Zwischenphase wurden verworfen; zu je 1 mL des Überstandes wurden 0.7 mL absoluter Ethanol gegeben und es wurde nochmals 1 Stunde bei 0°C inkubiert. Nach erneutem Zentrifugieren (gleiche Bedingungen wie oben) wurde der Überstand gegen Phosphatpuffer (20 mM $Na_2HPO_4$, pH 7.4, 150 mM NaCl) dialysiert. Schliesslich wurde die dialysierte Lösung gefriergetrocknet.

Das gleiche Verfahren wurde auch auf filtrierten Niederschlag B angewendet; im Extraktionspuffer wurde die Konzentration von Tris auf 110 mM erhöht. Gleiche Resultate wurden auch erzielt, wenn Chloroform durch Dichlormethan ersetzt wurde.

Herstellung von Niederschlag B: venöses, gepooltes Plasma wurde mit 4.8 M Na-Acetatpuffer (pH 4.0) auf pH 5.8 eingestellt; der Ethanolgehalt wurde auf 19% gebracht und gleichzeitig wurde die Temperatur von 0°C auf – 5.5°C gesenkt. Die Suspension wurde filtriert und der Überstand (Überstand a) für die Herstellung der Vorfällung IV weiter verwendet. Der Niederschlag (Niederschlag A) wurde in Wasser bei 0°C suspendiert (Proteinkonzentration: 2%), der pH-Wert mit einer Mischung von 1 Vol. 50 mM $Na_2HPO_4$ und 6 Vol. 50 mM Essigsäure auf 4.8 eingestellt und 2 Stunden gerührt. Danach wurde der pH-Wert durch Zugabe eines Puffers (1 Vol. 50 mM $Na_2HPO_4$ und 0.833 Vol. 50 mM Essigsäure) auf 5.1 eingestellt und die Ethanolkonzentration auf 12% erhöht, bei gleichzeitigem Absenken der Temperatur auf – 5°C. Durch Filtration dieser Suspension wurde der Niederschlag B gewonnen, der Überstand wurde weiter zur Isolierung von Immunglobulinen verwendet.

BEISPIEL 2

5 g Vorfällung IV [Kistler-Nitschmann Verfahren] wurde mit 20 mL Phosphatpuffer (20 mM $Na_2HPO_4$, 20 mM NaCl) versetzt; der pH wurde mit NaOH auf 7.0 eingestellt und die Suspension wurde während 30 Minuten bei 0°C inkubiert. Anschliessend wurde zentrifugiert (10 Minuten, 0°C, 10'000 × g) und zum Überstand Ethanol bis zu einer Endkonzentration von 68% zugegeben. Nach erneuter Inkubation (30 Minuten, 0°C) und Zentrifugation (10 Minuten, 0°C, 10'000 × g) wurde der Überstand wie in Beispiel 1 gegen Phosphatpuffer dialysiert und lyophilisiert.

Herstellung der Vorfällung IV: der Überstand a (siehe Beispiel 1) wurde weiter mit Ethanol versetzt bis zu einer Endkonzentration von 40% bei gleichzeitigem Absenken der Temperatur auf – 8°C. Aus dieser Suspension wurde durch Filtration die Vorfällung IV gewonnen, das Filtrat konnte weiter zur Isolierung von Albumin verarbeitet werden.

## BEISPIEL 3

1 g Niederschlag B aus einer Alkoholfraktionierung nach kistler-Nitschmann (filtriert, mit Filterhilfsmitteln) wurde mit 4 mL Puffer (entweder : a) 6 M Harnstoff, 10 mM Tris/HCl, pH 8.0 ; oder : b) 6 M Harnstoff, 20 mM $Na_2HPO_4$ ; oder : c) : 4 M Guanidin · HCl, 20 mM $Na_2HPO_4$) resuspendiert, und der pH-Wert mit 1 M NaOH auf 8.0 ± 0.2 eingestellt. Zur Suspension wurden 4 mL einer Mischung von Ethanol (96%) und Dichlormethan (1 Vol. Ethanol + 1 Vol. Dichlormethan) oder eine Mischung von Ethanol (96%) und Diethylether (1 Vol. Ethanol+3 Vol. Diethylether) gegeben, gemischt, und 1 Stunde bei 0°C stehen gelassen. Danach wurde zentrifugiert (10 Minuten, 10'000 × g, 0°C), die wässerige Phase mit einem gleichen Volumen Ethanol (96%) gemischt, 16 Stunden bei 4°C stehen gelassen und nochmals zentrifugiert (10 Minuten, 10'000 × g, 0°C). Der nach Zentrifugation erhaltene Überstand wurde 5 Stunden gegen 0.15 M NaCl bei 4°C dialysiert, nochmals zentrifugiert oder filtriert ; er enthielt danach das angereicherte Apolipoprotein A-I und konnte durch Dialyse oder Lyophilisation weiter verarbeitet werden.

Die aufgeführten Variationen oder beliebige Kombinationen davon führen zu praktisch demselben Endprodukt.

## BEISPIEL 4

1 g Niederschlag B (filtriert) wurde in 4 mL Puffer resuspendiert (Puffer : 6 M Harnstoff, 10 mM $H_3PO_4$, 10 mM $H_3BO_3$, 10 mM Zitronensäure). Der pH-Wert wurde mit 1 M NaOH auf 4.0 eingestellt und die Suspension 30 Minuten bei 20°C gerührt. Danach wurde 5 Minuten bei 3'000 × g, 20°C zentrifugiert, der Überstand auf 0°C abgekühlt und mit Ethanol versetzt (Endkonzentration 70%). Nach 1 Stunde wurde die Mischung zentrifugiert (15 Minuten, 4°C, 10'000 × g), und aus dem Überstand wurde nach Dialyse gegen PBS (mit Phosphat gepufferte Natriumchloridlösung ; 10 mM Phosphat, pH 7.4, 140 mM NaCl) über Nacht das gereinigte Apolipoprotein gewonnen.

## BEISPIEL 5

1 g Niederschlag B (filtriert) wurde in 4 mL Puffer (20 mM $Na_2HPO_4$) resuspendiert und der pH-Wert mit 1 M NaOH auf 8.0 eingestellt. Das Gemisch wurde nach 30 Minuten Rühren bei 0°C filtriert und das Filtrat langsam mit kaltem Ethanol versetzt bis zu einer Endkonzentration von 68%. Die Mischung wurde 1 Stunde bei 4°C stehen gelassen, dann wurde zentrifugiert (15 Minuten, 4°C, 10'000 × g), und aus dem Überstand nach 3 Stunden Dialyse gegen PBS bei 4°C und Filtration des Dialysats das angereicherte Apolipoprotein erhalten.

## BEISPIEL 6

Dem gemäss Beispiel 5 erhaltenen Überstand wurde pro Volumeneinheit entweder 0.1 bis 1 Volumen Wasser, 10 mM Phosphatpuffer pH 8.0, oder 10 mM Phosphatpuffer pH 8.0 mit NaCl bis zu 2 M zugegeben, die Mischung 30 Minuten bei 4°C gerührt und zentrifugiert. In diesem neuen Überstand fand sich das angereicherte Apolipoprotein, welches entweder durch Dialyse gegen eine Puffer mit tiefer Ionenstärke und anschliessender Lyophilisation, oder durch Präzipitieren mit Ethanol bis zu einer Endkonzentration von 78% aufkonzentriert werden konnte.

## BEISPIEL 7

1 g Vorfällung IV (filtriert, aus einer Alkoholfraktionierung nach dem Kistler-Nitschmann-Verfahren) wurde in 4 mL 20 mM NaCl oder in 4 mL Puffer (10 mM $H_3PO_4$, 10 mM $H_3BO_3$, 10 mM Zitronensäure) resuspendiert und der pH-Wert der NaCl-Suspension mit 1 M NaOH auf 8.15 eingestellt, derjenige der Suspension in Puffer auf pH 6.5 bis 7.5 Nach 1 Stunde Rühren bei 0°C wurde mit Ethanol gefällt (Endkonzentration 68%), zentrifugiert, und der Überstand mittels Dialyse und/oder einer weiteren Ethanolpräzipitation und Lyophilisation verarbeitet.

## BEISPIEL 8

45 kg Niederschlag B wurden in 180 L Pufferlösung (5 mM $NaHCO_3$, 68% Ethanol) suspendiert ; der pH-Wert wurde auf 7.2 eingestellt. Nach 3 Stunden Mischen wurde die Suspension bei 2°C filtriert. Der pH-Wert des Filtrates wurde auf 5.1 eingestellt ; nach Stehen über Nacht bei 1°C bildete sich ein Niederschlag, der ebenfalls abfiltriert wurde. Es wurden 557 g feuchter Niederschlag gewonnen, welcher durch Behandlung mit 96%igem Ethanol soweit möglich delipidiert und anschliessend eingefroren wurden. 100 g dieses Niederschlags wurden in 500 mL 4 M Guanidin-HCl, 5 mM Na-Phosphat pH 7.4 aufgelöst und 1 Stunde bei 20°C gerührt. Die Lösung wurde anschliessend filtriert, gegen 5 mM Na-Phosphatpuffer pH 7.4 diafiltriert und auf eine Proteinkonzentration von 6% gebracht.

## BEISPIEL 9

Die Lösung von Beispiel 8 wurde auf eine Endkonzentration von 10% Saccharose und eine Proteinkonzentration von 5% eingestellt, sterilfiltriert, in Glasflaschen abgefüllt und verschlossen, und 10 Stunden bei 60°C pasteurisiert.

BEISPIEL 10

Die Lösung von Beispiel 8 wurde auf eine Endkonzentration von 10% Saccharose oder 5% Mannit und eine Proteinkonzentration von 5% eingestellt, 10 Stunden bei 60°C pasteurisiert, steril in Glasflaschen zu je 50 mL abgefüllt, lyophilisiert und verschlossen. Vor Anwendung wurde das Lyophilisat in 50 mL sterilem, pyrogenfreiem Wasser aufgelöst.

BEISPIEL 11

100 g des nach Beispiel 8 gewonnen, eingefrorenen Niederschlags wurden in 500 mL 4 M Guanidin-HCl, 5 mM Na-Phosphat pH 7.4 aufgelöst und 1 Stunde bei 20°C gerührt. Die Lösung wurde anschliessend filtriert, gegen 20 mM Phosphatpuffer pH 7.4 dialysiert und weiter zu Liposomen verarbeitet:

a) Leicithin (77 g) und Cholesterin (11.6 g), beides aus einem organischen Lösungsmittel unter Stickstoff getrocknet, wurden mit Apolipoprotein (10 g) in Phosphatpuffer (10 L) und Na-Cholat (930 g) gemischt und 30 Minuten bei 24°C inkubiert. Mit einer anschliessenden Dialyse, Diafiltration, Gelchromatographie oder einer Inkubation mit einem Adsorbens wie BioBeads® (BioRad, Richmond, CA, U.S.A.) wurden die Liposomen vom Detergens abgetrennt. Anstatt Cholat konnten andere oberflächenaktive Substanzen wie z.B. Deoxycholat, Triton®, Brij®, Tween®, 3-[(3-cholamidopropyl)-dimethylammonio]-1-propansulfonat, oder Octyl-β-Glucosid in geeigneten Mengen eingesetzt werden.

b) In einem organischen Lösungsmittel wie z.B. Ethanol oder Ether gelöste Lipide wurden durch eine feine Nadel in die Proteinlösung eingespritzt ; das Lösungsmittel wurde anschliessend durch eine der unter a) erwähnten Methoden oder durch Erwärmen des Gemisches wieder entfernt.

c) Lipide wurden als Emulsion zu der Proteinlösung gegeben und mit einer Ultraschall-Sonde oder einem Ultraschall-Bad unter Sauerstoff-Ausschluss beschallt. Die Behandlung mit Ultraschall konnte mit Einfrieren/Auftauen des Gemisches kombiniert werden.

Die unter a), b) und c) beschriebenen Produkte konnten als Proteoliposomen oder als Protein-Lipid-Gemische in flüssiger oder lyophilisierter Form zur Anwendung gebracht werden.

In den Beispielen 1 bis 11 können den Pufferlösungen Zusätze zum Schutz vor Oxidation (z.B. 1 mM Mercaptoethanol, Dithiothreitol, Glutathion oder EDTA) oder Proteaseaktivität (synthetische oder natürliche Protease-Inhibitoren) beigegeben werden.

Dem Endprodukt können zudem zur Stabilisierung und Erhöhung der biologischen Aktivität Lipide (z.B. Lecithin) zugesetzt werden, und zur Verbesserung der Löslichkeit des Lyophilisats Zucker (z.B. 5 bis 10% Saccharose oder Mannit), Glycin (0,1 bis 0,5 M) oder Albumin (1 bis 10 g/L Lösung).

Dies sind lediglich Beispiele ; der Fachmann kann leicht weitere Varianten aufstellen, ohne den Schutzbereich des Patents zu verlassen.

**Patentansprüche**

1. Verfahren zur Herstellung von Apolipoproteinen aus Fraktionen von menschlichem Blutplasma oder Serum, welche Lipoproteine enthalten, dadurch gekennzeichnet, dass man besagte Fraktionen in einer wässerigen Pufferlösung im pH-Bereich 3 bis 5 oder 6 bis 9, bzw. 3 bis 9, wenn unter Zusatz von chaotropen Stoffen, Tensiden oder wasserlöslichen Salzen gearbeitet wird, suspendiert und zur Ausfällung von unerwünschten Begleitstoffen mit einem niederen aliphatischen Alkohol inkubiert, die Lipoproteine enthaltende Phase durch Filtrieren, Zentrifugieren oder Dekantieren abtrennt, auf einen höheren Gehalt an Lipoproteinen bringt und, wenn das Produkt dabei als Niederschlag erhalten wird, diesen Lipoproteinniederschlag auflöst.

2. Verfahren nach Anspruch 1, dadurch gekennzeichnet, dass man die Suspension in der wässerigen Pufferlösung zuerst filtriert, zentrifugiert oder dekantiert und erst dann das Filtrat bzw. den Überstand mit dem niederen aliphatischen Alkohol inkubieren lässt.

3. Verfahren nach Anspruch 1, dadurch gekennzeichnet, dass man mit dem niederen aliphatischen Alkohol in der Kälte inkubiert.

4. Verfahren nach Anspruch 1, dadurch gekennzeichnet, dass man die Lipoproteine enthaltende Phase durch Membranfiltration oder Lyophilisation oder durch Ausfällung bei pH 5 bis 6 bzw. mittels Zusatzes eines niederen aliphatischen Alkohols, eines wasserlöslichen Salzes oder eines polymeren Stoffes, oder durch Adsorption an und Elution von einem festen Trägermaterial auf einen höheren Gehalt an Lipoproteinen bringt.

5. Verfahren nach Anspruch 1, dadurch gekennzeichnet, dass man den Lipoproteinniederschlag durch leichtes Erwärmen oder durch Zusatz von chaotropen Stoffen oder Tensiden oder einer schwach basischen Pufferlösung auflöst und den Zusatz oder die Zusätze durch Dialyse, Diafiltration, Adsorption oder Gelfiltration entfernt.

6. Verfahren nach einem der Ansprüche 1 bis 5, dadurch gekennzeichnet, dass man die abgetrennte, die Lipoproteine enthaltende Phase, bevor sie auf einen höheren Gehalt gebracht wird, zur weiteren Reinigung gegen eine wässerige Pufferlösung von pH 3 bis 5 oder 6 bis 9 dialysiert und die dialysierte Lösung gewinnt.

7. Verfahren nach einem der Ansprüche 1 bis 5, dadurch gekennzeichnet, dass man die abgetrennte, die Lipoproteine enthaltend Phase, bevor sie auf einen höhreren Gehalt gebracht wird, zur weiteren Reinigung mit einer wässerigen Pufferlösung mit einem pH-Wert um 8 versetzt,den sich bildenden Niederschlag entfernt und die flüssige Phase gewinnt.

8. Verfahren nach einem der Ansprüche 1 bis 7, dadurch gekennzeichnet, dass man die Suspension in der wässerigen Pufferlösung mit dem niedrigen aliphatischen Alkohol in Mischung mit einem apolaren organischen Lösungsmittel inkubiert, wodurch ein Mehrphasensystem entsteht, von welchem man die wässerige Phase abtrennt und nochmals mit einem niedrigen aliphatischen Alkohol inkubiert.

9. Verfahren nach einem der Ansprüche 1 bis 8, dadurch gekennzeichnet, dass man ausserdem zur Entfernung der Lipide das Produkt auf einer beliebigen Stufe mit einem organischen Lösungsmittel behandelt.

10. Verfahren zur Herstellung pharmazeutischer Präparate zur Prophylaxe und Therapie von kardiovaskulären Krankheiten, dadurch gekennzeichnet, dass man gemäss Ansprüchen 1 bis 9 Apolipoproteine herstellt und die erhaltenen Apolipoproteine mit üblichen Trägerstoffen und Excipientien zu pharmazeutischen Präparaten verarbeitet.

11. Verfahren nach Anspruch 10, dadurch gekennzeichnet, dass man die erhaltenen Apolipoproteine mit Lipiden zu Proteoliposomen oder Protein-Lipid-Gemischen in flüssiger oder lyophilisierter Form verarbeitet.

## Claims

1. A process of preparation of apolipoproteins from fractions of human blood plasma or serum which contain lipoproteins, which comprises suspending said fractions in an aqueous buffer solution of pH range 3 to 5 or 6 to 9 and of pH range 3 to 9, respectively, if working in the presence of chaotropic substances, surface-active substances or water-soluble salts, and incubating with a lower aliphatic alcohol for precipitation of undesirable contaminants, separating the lipoproteins containing phase by filtration, centrifugation or decantation, bringing it to a higher concentration of lipoproteins and if the product thereby is obtained as a precipitate, dissolving said lipoprotein precipitate.

2. The process according to claim 1, wherein the suspension in the aqueous buffer solution is first filtered, centrifuged or decanted, before the filtrate or supernatant is incubated with the lower aliphatic alcohol.

3. The process according to claim 1, wherein the incubation with the lower aliphatic alcohol is carried out in the cold.

4. The process according to claim 1, wherein the lipoproteins containing phase is brought to a higher concentration of lipoproteins by membrane filtration, lyophilization, precipitation at pH 5 to 6, or by addition of a lower aliphatic alcohol, a water-soluble salt or a polymeric substance, or by adsorption to and elution from a solid carrier material.

5. The process according to claim 1, wherein the lipoprotein precipitate is dissolved by moderate heating or by adding chaotropic substances, surface-active substances, or a slightly basic buffer solution, said substances then being removed by dialysis, diafiltration, adsorption or gel filtration.

6. The process according to any one of claims 1 to 5, wherein the separated, lipoproteins containing phase for further purification is dialyzed against an aqueous buffer solution of pH range 3 to 5 or 6 to 9 before the lipoproteins are brought to a higher concentration, and the dialyzed solution is recovered.

7. The process according to any one of claims 1 to 5, wherein the separated, lipoproteins containing phase for further purification is treated with an aqueous buffer solution of pH about 8 before the lipoproteins are brought to a higher concentration, the thus formed precipitate is discarded and the liquid phase is recovered.

8. The process according to any one of claims 1 to 7, wherein the suspension in the aqueous buffer solution is incubated with the lower aliphatic alcohol in admixture with a non-polar organic solvent, thereby causing the formation of a multi-phase system from which the aqueous phase is separated and incubated again with the lower aliphatic alcohol.

9. The process according to any one of claims 1 to 8, wherein the product, in addition, is treated at any stage with an organic solvent for removal of the lipids.

10. A process for the manufacture of pharmaceutical compositions intended for the prophylaxis and therapy of cardiovascular diseases, which comprises preparing apolipoproteins according to claims 1 to 9 and working up the obtained apolipoproteins with usual carrier substances and excipients to pharmaceutical compositions.

11. A process according to claim 10, which comprises working up the obtained apolipoproteins with lipids to proteoliposomes or protein-lipid mixtures in liquid or in lyophilized form.

## Revendications

1. Procédé de préparation d'apolipoprotéines à partir de fractions de plasma sanguin ou de sérum humain qui contiennent des lipoprotéines, caractérisé en ce que l'on suspend lesdites fractions dans une solution aqueuse d'un tampon ayant une plage de pH de 3 à 5 ou de 6 à 9, respectivement de 3 à 9 lorsqu'on travaille avec adjonction de substances chaotropes, de substances tensioactives ou de sels hydrosolubles, et l'on fait incuber avec un alcool aliphatique inférieur pour précipiter des produits secondaires indésirables, on sépare la phase qui contient les lipoprotéines par filtration, centrifugation ou décantation, on l'amène à une teneur plus élevée en lipoprotéines et, lorsque le produit est obtenu alors sous forme d'un précipité, on dissout ce précipité de lipoprotéines.

2. Procédé selon la revendication 1, caractérisé en ce que l'on filtre, centrifuge ou décante tout d'abord la suspension dans la solution aqueuse de tampon et que l'on fait incuber le filtrat ou le liquide surnageant avec l'alcool aliphatique inférieur seulement ensuite.

3. Procédé selon la revendication 1, caractérisé en ce que l'on fait incuber avec l'alcool aliphatique inférieur à froid.

4. Procédé selon la revendication 1, caractérisé en ce que l'on amène la phase qui contient les lipoprotéines à une teneur plus élevée en lipoprotéines par filtration à travers une membrane ou par lyophilisation ou par précipitation à un pH de 5 à 6 ou au moyen d'une adjonction d'un alcool aliphatique inférieur, d'un sel hydrosoluble ou d'une substance polymère, ou par adsorption à et élution à partir d'une matière de support solide.

5. Procédé selon la revendication 1, caractérisé en ce que l'on dissout le précipité de lipoprotéines en chauffant légèrement ou en ajoutant des substances chaotropes ou des substances tensioactives ou une solution d'un tampon faiblement basique et on élimine l'adjonction ou les adjonctions par dialyse, diafiltration, adsorption ou filtration sur un gel.

6. Procédé selon l'une des revendications 1 à 5, caractérisé en ce que l'on dialyse la phase séparée qui contient les lipoprotéines, aux fins d'une purification plus poussée, avant qu'elle soit amenée à une teneur plus élevée, contre une solution aqueuse d'un tampon de pH 3 à 5 ou 6 à 9 et l'on recueille la solution dialysée.

7. Procédé selon l'une des revendications 1 à 5, caractérisé en ce que l'on traite la phase séparée qui contient les lipoprotéines, aux fins d'une purification plus poussée, avant qu'elle soit amenée à une teneur plus élevée, par une solution aqueuse d'un tampon d'une valeur de pH d'environ 8, on élimine le précipité qui s'est formé et on recueille la phase liquide.

8. Procédé selon l'une des revendications 1 à 7, caractérisé en ce que l'on fait incuber la suspension dans la solution aqueuse d'un tampon avec l'alcool aliphatique inférieur en mélange avec un solvant organique non polaire, ce par quoi se forme un système de plusieurs phases dont on sépare la phase aqueuse et on la fait incuber encore une fois avec un alcool aliphatique inférieur.

9. Procédé selon l'une des revendications 1 à 8, caractérisé en ce que, en outre, on traite le produit à une étape quelconque par un solvant organique, afin d'éliminer les lipides.

10. Procédé de préparation de compositions pharmaceutiques destinées au traitement prophylactique et thérapeutique de maladies cardio-vasculaires, caractérisé en ce que l'on prépare des apolipoprotéines selon les revendications 1 à 9 et on transforme les apolipoprotéines obtenues en compositions pharmaceutiques à l'aide de matières de support et d'excipients usuels.

11. Procédé selon la revendication 10, caractérisé en ce que l'on transforme les apolipoprotéines obtenues, avec des lipides, en protéoliposomes ou en mélanges de protéines et de lipides sous forme liquide ou lyophilisée.